# FASCICULE DE BREVET EUROPEEN

(11) **EP 0 850 039 B1**
(45) Date de publication et mention de la délivrance du brevet: **25.07.2001**
(21) Numéro de dépôt: 95931252.1
(22) Date de dépôt: 15.09.1995
(51) Int. Cl.: A61K 7/00

(54) **SAVON ANTISEPTIQUE LIQUIDE**
FLÜSSIGE ANTISEPTISCHE SEIFE
LIQUID ANTISEPTIC SOAP

(43) Date de publication de la demande: 01.07.1998
(73) Titulaire: Rothan, Gabriel, 77330 Ozoir-la-Ferrière (FR)
(72) Inventeur: Rothan, Gabriel, 77330 Ozoir-la-Ferrière (FR)
(74) Mandataire: Rhein, Alain
(86) Numéro de dépôt international: FR9501188
(87) Numéro de publication internationale: WO9709957

(56) Documents cités:
- WO-A-91/06277
- WO-A-95/11661
- US-A- 4 828 542
- SEIFEN-ÖLE-FETTE-WACHSE, vol. 12, 20 Juin 1974, pages 293-294, XP002002993 J. VERONESE:

## Description

L'invention concerne un savon antiseptique liquide notamment destiné au lavage des mains du personnel soignant.

La présente invention trouvera son application, plus particulièrement, auprès des fabriquants de savon antiseptique, notamment liquide, utilisé d'une façon générale, par le personnel soignant.

Il est à présent établi que la transmission manuportée est la cause essentielle des infections nosocomiales. Aussi, le lavage des mains doit être convenablement effectué que ce soit avant un geste médical invasif ou après un soin très contaminant.

A ce propos, de nombreuses études bactériologiques ont pu démontrer que la durée de lavage des mains correspond au paramètre, probablement le plus influent sur la réduction significative du nombre de germes transportés. Ainsi, de nombreux travaux dans le domaine ont permis de conclure qu'un temps de lavage de une à deux minutes permettait d'obtenir la garantie de l'antisepsie.

Bien que ce temps paraisse relativement court, il est rarement respecté par le personnel soignant, d'abord en raison de la pression qu'il subit du fait d'une surcharge systématique de travail. Se pose également la difficulté de prendre en considération l'évolution réelle du temps au cours de l'exécution d'une opération aussi naturelle que le lavage des mains qui, par ailleurs, doit être répété de nombreuses fois au cours d'une journée.

Ainsi, si ce personnel soignant est conscient de l'utilité de cette opération, il ne dispose pas toujours de moyens de mesure du temps lui permettant de fournir l'indication que le résultat recherché, à savoir la garantie d'antisepsie, est obtenue. De toute manière, activer un quelconque dispositif indicateur du temps de lavage est ressenti comme une contrainte supplémentaire, sans compter que, dans l'absolu, chacun s'estime capable d'apprécier le temps écoulé, tout particulièrement, lorsqu'il s'agit d'une voire deux minutes.

Or, précisément, ce temps de lavage n'est quasiment jamais respecté tel qu'a pu le démontrer un nombre non négligeable d'observations réalisées en milieu hospitalier.

Il convient d'observer, qu'il est connu, par le document SEIFEN-ÖLE-FETTE-WACHSE, vol. 12, du 20.06.74, pages 293-294, XP002002993, l'utilisation de microcapsules dans des produits alimentaires, dans les cosmétiques ou encore dans les produits de lavage. Toutefois, ce document n'apporte pas de solution au problème posé.

La présente invention se veut, par conséquent, à même d'apporter une solution au problème précité, c'est-à-dire prévenir la transmission manuportée, cause essentielle des infections nosocomiales, ceci en donnant au personnel soignant des moyens d'indication précis quant à l'écoulement du temps de lavage des mains et, donc, de l'obtention de la garantie d'avoir atteint le degré d'antisepsie souhaité. Finalement, cette indication, du type visuel et son obtention sont directement liées à l'action du lavage des mains, sans qu'il soit nécessaire de faire appel à des moyens annexes qu'il convient d'activer indépendamment.

A cet effet, l'invention concerne un savon antiseptique liquide, notamment destiné au lavage des mains du personnel soignant, caractérisé par le fait que dans sa composition sont ajoutés des moyens de coloration qui, par adjonction d'eau ou d'un réactif au savon, soit, voient leur pouvoir colorant activé ou désactivé, soit, libèrent leur principe actif ou encore agissent sous l'influence de la variation du pH, ceci dans le but de modifier la teinte de ce savon au-delà d'un temps de lavage déterminé.

En fait, le principe consiste à mélanger aux composants habituels du savon antiseptique, préférentiellement sous forme liquide, des moyens de coloration à même de modifier la couleur de ce savon lorsque, précisément, l'usager s'est suffisamment frotté les mains et, donc, qu'il a atteint le degré d'antisepsie souhaité.

Dans ces conditions, ce personnel soignant est dispensé de se préoccuper du temps réellement attribué pour le lavage des mains avant un geste médical invasif ou après un soin très contaminant, puisque l'usage même du savon est en mesure de donner cette indication de manière visuelle.

En outre, l'usage d'un tel savon aura tendance à donner mauvaise conscience à l'usager ne respectant pas un temps de lavage suffisant pour constater la variation de la teinte du savon. Aussi, l'on peut estimer que même les plus pressés en raison d'un manque de temps évident en viendront à respecter cette condition d'hygiène primordiale que représente le lavage des mains jusqu'à l'obtention de la garantie d'antisepsie de sorte que l'on puisse enfin réduire, de manière significative, les infections nosocomiales dues à des transmissions manuportées.

Différents modes de réalisation de la présente invention vont être présentés dans la description qui va suivre dont la compréhension sera facilitée en se référant au dessin ci-joint dans lequel :
- la figure 1 est une vue schématisée d'un distributeur de savon liquide antiseptique contenant, en tant que moyens de coloration, un indicateur coloré ;
- la figure 2 est une vue schématisée d'un distributeur de savon liquide antiseptique contenant des moyens de coloration sous forme d'un colorant agissant sous l'influence d'un réactif s'écoulant d'un second distributeur ;
- les figures 3 à 5 illustrant un premier procédé d'obtention de microsphères contenant en tant que principe actif, un colorant et constituant lesdits moyens de coloration ;
- les figures 6 et 7 illustrent un second procédé d'obtention de ces microsphères constituant les moyens de coloration destinés à être mélangés à la composition du savon liquide antiseptique ;
- la figure 8 est une vue schématisée d'un distributeur de savon liquide antiseptique contenant en tant que moyens de coloration, des microsphères.

La présente invention est relative à un savon antiseptique qui est plus particulièrement destiné au personnel soignant pour le lavage de leurs mains, notamment avant un acte médical invasif par exemple. En fait, le personnel soignant doit être tout particulièrement attentif au temps alloué à ce lavage de leurs mains dans la mesure où, précisément, le respect de ce facteur temps est primordial pour obtenir une réduction significative du nombre de germes habituellement responsables des contaminations par transmission manuportée.

En fait, selon l'invention, ce savon antiseptique est caractérisé en ce que, dans sa composition, sont ajoutés les moyens de coloration qui, par adjonction d'eau ou d'un réactif au savon soit voient leur pouvoir colorant activé ou désactivé, soit libèrent leurs principes actifs ou encore agissent sous l'influence de la variation du pH que produit, par exemple, l'adjonction d'eau, ceci dans le but de modifier la teinte de ce savon au-delà d'un temps de lavage déterminé.

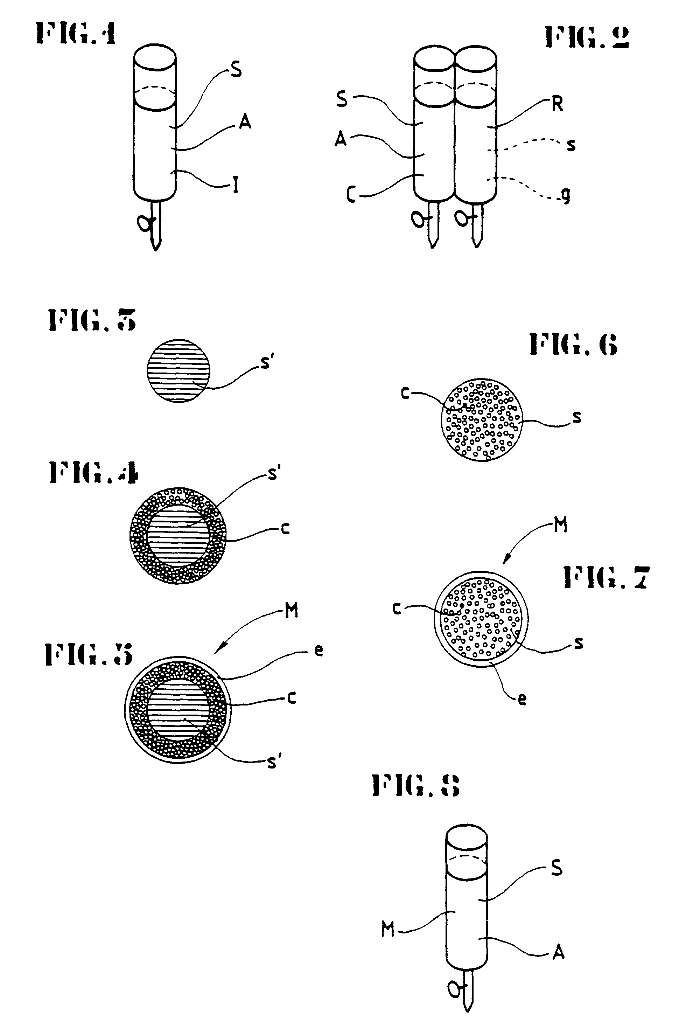

Ainsi, selon un premier mode de réalisation correspondant à la figure 1, ces moyens de coloration peuvent se présenter sous forme d'indicateur coloré I ajouté à la composition du savon S et qui possède une zone de virage compatible avec le pH. En réalité, si le pH d'un savon antiseptique, notamment liquide, est d'environ 6,5, il y a lieu de tenir compte, tout particulièrement, de la plage du pH dans laquelle le composé antiseptique A exerce son pouvoir antimicrobien, ceci de manière optimale.

A ce propos, il est pris comme exemple préférentiel de composé antiseptique A, la chlorhexidine ayant un pouvoir antimicrobien optimum entre un pH de 5,5 à 7.

Ainsi, il peut être retenu en tant qu'indicateur coloré compatible avec le résultat recherché :
- le rouge de phénol (C19 H 1405, S) ;
- le rouge de bromophénol (C19 H11 Na05, S) ;
- le bleu de bromothymol (C27 H 18 Br205) ;
- indicateur universel.

Le temps de virage est dépendant, principalement, de l'écart de pH existant entre la solution du savon à l'état pur et la solution d'utilisation, c'est-à-dire avec adjonction d'eau.

On notera que les exemples d'indicateur coloré mentionnés ci-dessus ne correspondent pas à une liste limitative de la présente invention et il est bien entendu possible de faire appel à d'autres types d'indicateurs colorés dès l'instant qu'ils répondent aux différentes exigences. Plus particulièrement, il peut, notamment, être souhaité que cet indicateur coloré figure sur la liste des colorants utilisés en application cosmétologique. Par ailleurs, il doit avoir un comportement neutre à l'égard des caractéristiques physico-chimiques du composé antiseptique.

Selon un autre mode de réalisation correspondant à la figure 2, les moyens de coloration sont constitués par un colorant C intégré à la composition du savon liquide S, lequel colorant est à même de modifier la couleur de ce savon sous l'influence d'un réactif R préférentiellement contenu dans un deuxième récipient et que l'on vient additionner au savon liquide S contenant l'antiseptique A au début du lavage des mains. En réalité, ce réactif R aura pour conséquence de colorer ou décolorer, progressivement, le savon, ceci au-delà d'un temps de lavage défini, donnant à l'usager l'indication précise du temps de lavage à respecter.

Les exemples de colorants susceptibles d'être retenus sont communiqués ci-dessous.

Il s'agit :
- du bleu patente V identifié sous le n° E 131 selon la réglementation européenne, CI 42051, lequel colorant réagit sous l'influence de l'acide ascorbique C6H808 en solution à environ 1 gramme/litre produisant une variation de couleur de bleu à vert émeraude
- le rouge 22, CI 45350, de formule C20H605Na2,Br4, le réactif correspondant à une solution à 5 % de sulfate de fer FeS04 induisant une variation de couleur du rouge fluorescent au noir ; il peut encore être utilisé un réactif différent correspondant à une solution à 5 % d'alun de potasse K08S2A16H20 induisant une variation de couleur du rouge fluorescent au jaune
- le jaune n° 7, CI10316 de formule C20H1205, utilisé en combinaison avec le réactif correspondant à une solution à 5 % de sulfate de fer FeS04 induisant une variation de couleur du jaune fluorescent au noir ;
- l'indigotine ou bleu n° 2 référencé E132 selon la réglementation européenne, CI 73015 de formule C16H8N208S2Na2, avec comme réactif une solution à 10 % de bicarbonate de sodium conduisant à une décoloration totale du bleu initial ;
- le vert n° 3 lequel peut être utilisé en combinaison avec le réactif correspondant à une solution à 5 % de sulfate de fer FeS04, induisant une variation de couleur du vert au noir ;
- le vert n° 8, CI59040 avec une solution à 5 % de sulfate de fer FeS04 comme réactif, induisant une variation de couleur du vert au noir.

Là encore, il convient de prendre en considération le fait que l'énumération des colorants ci-dessus n'est nullement limitative et l'on peut, naturellement, imaginer l'usage d'autre types de colorants à même de répondre aux besoins de l'invention.

Il est évident que ce second mode de réalisation nécessite le conditionnement du savon antiseptique (S,A) et du réactif R dans des flacons distincts. A ce propos, le réactif R peut être mélangé, là encore, avec du savon s, et/ou une solution adoucissante glycérinée g dépourvue de colorant. De toute manière, ces flacons seront conditionnés, préférentiellement, de telle manière à rendre leur usage commode pour le personnel soignant qui est amené à se laver les mains de nombreuses fois au courant d'une journée.

Selon un troisième mode de réalisation, correspondant aux figures 3 à 7, les moyens de coloration se présentent sous forme de microsphères qui libèrent leur principe actif (soit un colorant) au-delà d'un temps déterminé sous l'adjonction d'eau au savon, soit par un phénomène de dissolution simple soit sous l'influence d'une variation de pH issue, précisément, de cette adjonction d'eau.

En fait, ces microsphères M se présentent sous forme de minuscules sphères s, s', soit contenant le colorant en question, soit constituant, simplement, un support neutre lequel est, ensuite, enveloppé du principe actif Pa que constitue ce colorant. Au cours de l'opération suivante, ces minuscules sphères s, s' sont enrobées d'une pellicule e constituée par un produit susceptible de se perméabiliser sous l'influence d'une variation du pH issu de l'adjonction d'eau au savon ou encore apte à se dissoudre, progressivement, dans cette eau. Finalement, ce n'est qu'après la perméabilisation de cette pellicule e ou sa dissolution que le colorant est à même de modifier la couleur du savon.

Ainsi, le principe de la fabrication des microgranules M faisant appel à des supports neutres s' sur lesquels sont fixés, ultérieurement, le ou les principes actifs C, s'apparente à la dragéification en turbine. Le choix du cristal d'amorce et sa forme cristalline sont les paramètres importants pour l'obtention d'un microgranule M sphérique dont le diamètre peut varier de 0,4 à 1 mm.

A côté de cela il existe le procédé de fabrication dit d'extrusion et sphéronisation qui consiste à extruder une masse humide d'ingrédients qui donne un extrudé puis à transformer celui-ci en de fines parties sphériques. Comme on le conçoit, l'avantage d'une telle solution permet d'additionner à la masse humide d'ingrédients destinés à constituer le support neutre le principal actif correspondant au colorant C. En fait, de telles microsphères ainsi obtenues sont à même de contenir un taux plus élevé de colorant.

Quoi qu'il en soit, ces microgranules M, (quel que soit leur mode d'obtention), chargées du principe actif, sont destinées à l'enrobage qui consiste à déposer sur ces microgranules une membrane de polymère e, plus ou moins épaisse, de propriété physico-chimique connue du point de vue de sa structure et de sa solubilité et qui contrôle la libération du principe actif.

A titre d'exemple, l'enrobage e peut être réalisé au moyen d'acetylphtalate de cellulose qui a pour avantage de libérer le colorant dans le savon sous l'influence d'une variation du pH se situant dans la plage compatible avec celle, précisément, du savon. Ainsi, celui-ci peut avoir un pH de l'ordre de 5,5 à l'origine, ce pH augmentant, progressivement, par adjonction d'eau pour atteindre une valeur supérieure à 6 conduisant à la perméabilisation de l'enrobage e. Il existe, là encore, de multiples matériaux susceptibles de constituer un tel enrobage e et l'exemple ci-dessus n'est donné qu'à titre indicatif. Ainsi, il existe des polymères de chacun des trois principaux groupes chimiques (vinylique, cellulosique et acrylique) à même de répondre aux besoins de la présente invention. Certains nécessitent l'emploi d'un diluant organique tandis que d'autres sont susceptibles d'être mis en oeuvre en milieu aqueux.

On observera, tout particulièrement, que cette technologie des microsphères est particulièrement avantageuse dans la mesure où elle permet d'intervenir sur plusieurs paramètres afin d'obtenir le résultat recherché, c'est-à-dire la libération du principe actif au-delà d'un temps de lavage donné. Tout particulièrement, l'on peut tout d'abord intervenir sur la nature de la pellicule correspondant à l'enrobage et son épaisseur en fonction du temps de libération souhaité pour le colorant. Il existe, également, moins de contraintes en ce qui concerne le choix de ce dernier. Ainsi, il n'est pas indispensable que ce colorant varie de teinte en fonction du pH. Aussi, il sera plus aisé pour le fabriquant de retenir un produit qui soit neutre, vis-à-vis des caractéristiques physico-chimiques du savon ou encore du produit antiseptique ainsi qu'à l'égard de l'activité antiseptique, de ce dernier.

Finalement, selon un quatrième mode de réalisation, ces moyens de coloration peuvent se présenter sous forme de liposomes correspondant à des micelles de lipides formant des capsules dans lesquelles peuvent être introduites toutes sortes de molécules et, notamment, des colorants.

Le savon antiseptique, objet de la présente invention, vient, ainsi, répondre, de manière avantageuse, aux besoins ressentis par le personnel soignant conscient du risque que représente la transmission manuportée.

## Revendications

1. Savon antiseptique liquide, notamment destiné au lavage des mains du personnel soignant, caractérisé par le fait que dans sa composition sont ajoutés des moyens de coloration qui, par adjonction d'eau ou d'un réactif au savon, soit voient leur pouvoir colorant activé ou désactivé, soit libèrent leur principe actif ou encore agissent sous l'influence de la variation du pH, ceci dans le but de modifier la teinte de ce savon au-delà d'un temps de lavage déterminé.

2. Savon antiseptique liquide selon la revendication 1, caractérisé par le fait que les moyens de coloration se présentent sous forme d'un indicateur coloré (I) ajouté à la composition du savon (S) et qui possède une zone de virage compatible avec le pH de ce dernier.

3. Savon antiseptique liquide selon la revendication 2, caractérisé par le fait que l'indicateur coloré est soit le rouge de phénol, soit le rouge de bromophénol, soit le bleu de bromothymol, soit l'indicateur universel.

4. Savon antiseptique liquide selon la revendication 1, caractérisé par le fait que les moyens de coloration sont constitués par un colorant (C) intégré dans la composition du savon liquide (S), lequel colorant est à même de modifier la couleur de ce savon sous l'influence d'un réactif (R) au-delà d'un temps de lavage défini.

5. Savon antiseptique liquide selon la revendication 4, caractérisé par le fait que le réactif (R) est contenu dans un récipient différent de celui contenant le savon liquide (S) antiseptique.

6. Savon antiseptique liquide selon la revendication 5, caractérisé par le fait que le réactif (R) est mélangé, soit avec du savon (s) et/ou une solution adoucissante glycérinée (g) dépourvue de colorant.

7. Savon antiseptique liquide selon la revendication 1, caractérisé par le fait que les moyens de coloration se présentent sous forme de microsphères (M) à même de libérer un colorant au-delà d'un temps déterminé sous l'adjonction d'eau au savon, soit par un phénomène de dissolution simple, soit sous l'influence d'une variation de pH issu de ladite adjonction d'eau.

8. Savon antiseptique liquide selon la revendication 7, caractérisé par le fait que les microsphères (M) se présentent sous forme de minuscules sphères (s') contenant le colorant, ces minuscules sphères (s') étant enrobées d'une pellicule (e) constituée un produit susceptible de se perméaliser sous l'influence d'une variation du pH issue de l'adjonction d'eau au savon ou encore apte à se dissoudre, progressivement, dans cette eau.

9. Savon antiseptique liquide selon la revendication 7, caractérisé par le fait que les microsphères (M) se présentent sous forme de minuscules sphères (s) constituant un support neutre enveloppé du principe actif (c) que constitue le colorant, puis enrobé d'une pellicule (e) constituée par un produit susceptible de se perméabiliser sous l'influence d'une variation du pH issue de l'adjonction d'eau au savon ou encore apte à se dissoudre, progressivement dans cette eau.

10. Savon antiseptique liquide selon l'une quelconque des revendications 8 et 9, caractérisé par le fait que l'enrobage (e) est réalisé au moyen d'acetylphtalate de cellulose.

11. Savon antiseptique liquide selon la revendication 1, caractérisé par le fait que des moyens de coloration se présentent sous forme de liposomes formant des capsules dans lesquelles sont introduites des colorants.

## Patentansprüche

1. Flüssige antiseptische Seife, nämlich zum Waschen der Hände des Pflegepersonals, dadurch gekennzeichnet, daß ihrer Zusammensetzung Färbemittel zugefügt werden, die durch Zufügung von Wasser oder einem Reagens für Seife entweder deren Färbekraft erregt oder entregt sehen oder ihren Wirkstoff freigeben oder sogar unter dem Einfluß der pH-Änderung wirksam werden, und zwar um die Farbe dieser Seife nach einer bestimmten Waschzeit zu ändern.

2. Flüssige antiseptische Seife nach Anspruch 1, dadurch gekennzeichnet, daß die Färbemittel als ein gefärbter Indikator (I) ausgestaltet sind, der der Zusammensetzung der Seife (s) zugefügt wird und einen mit dem pH-Wert der letzten vereinbaren Wendebereich hat.

3. Flüssige antiseptische Seife nach Anspruch 2, dadurch gekennzeichnet, daß der gefärbte Indikator entweder Phenolrot oder Bromphenolrot oder Bromthymolblau oder der Universalindikator ist.

4. Flüssige antiseptische Seife nach Anspruch 1, dadurch gekennzeichnet, daß die Färbemittel aus einem in der Zusammensetzung der flüssigen Seife (s) integrierten Fabrstoff (c) bestehen, welcher Fabrstoff fähig ist, die Farbe dieser Seife unter dem Einfluß eines Reagenses (R) nach einer bestimmten Waschzeit zu ändern.

5. Flüssige antiseptische Seife nach Anspruch 4, dadurch gekennzeichnet, daß das Reagens (R) in einem Behälter enthalten ist, der von demjenigen, der die flüssige antiseptische Seife (s) enthält, unterschiedlich ist.

6. Flüssige antiseptische Seife nach Anspruch 5, dadurch gekennzeichnet, daß das Reagens (R) entweder der Seife (s) und/oder einer farbstoffreien, glyzerinhaltigen Erweichungslösung (g) zugemischt wird.

7. Flüssige antiseptische Seife nach Anspruch 1, dadurch gekennzeichnet, daß die Färbemittel als Mikrokugeln (M) ausgestaltet sind, die geeignet sind, unter der Zufügung von Wasser zu der Seife, entweder zufolge eines einfachen Lösungsphänomens oder unter dem Einfluß einer sich aus der genannten Wasserzufügung ergebenden pH-Änderung, nach einer bestimmten Zeit einen Farbstoff freizugeben.

8. Flüssige antiseptische Seife nach Anspruch 7, dadurch gekennzeichnet, daß die Mikrokugeln (M) als ganz kleine, den Farbstoff enthaltende Kugeln (s') ausgestaltet sind, wobei diese ganz kleinen Kugeln (s') mit einem Film (e) überzogen sind, der aus einem Produkt besteht, das unter dem Einfluß einer sich aus der Zufügung von Wasser zu der Seife ergebenden pH-Änderung durchlässig werden kann oder geeignet ist, sich in diesem Wasser allmählich zu lösen.

9. Flüssige antiseptische Seife nach Anspruch 7, dadurch gekennzeichnet, daß die Mikrokugeln (M) als ganz kleine Kugeln (s) ausgestaltet sind, die einen neutralen Träger bilden, der mit dem durch den Farbstoff gebildeten Wirkstoff, anschließend mit einem Film (e) überzogen wird, der aus einem Produkt besteht, das unter dem Einfluß einer sich aus der Zufügung von Wasser zu der Seife ergebenden pH-Änderung durchlässig werden kann oder geeignet ist, sich in diesem Wasser allmählich zu lösen.

10. Flüssige antiseptische Seife nach irgendeinem der Ansprüche 8 oder 9, dadurch gekennzeichnet, daß die Umhüllung (e) mittels Zelluloseazetylphthalat erfolgt.

11. Flüssige antiseptische Seife nach Anspruch 1, dadurch gekennzeichnet, daß die Färbemittel als Liposome ausgestaltet sind, die Kapseln bilden, in die Farbstoffe eingeführt werden.

## Claims

1. Liquid antiseptic soap, namely for the nursing staff to wash its hands, characterised in that to its composition are added colouring means that, through the adjunction of water or a reagent to soap, either have their colouring value activated or deactivated, or release their active constituent or even react under the influence of the change in pH, this in order to change the colour of the soap after a determined period of washing.

2. Liquid antiseptic soap according to claim 1, characterised in that the colouring means are in the form of a coloured indicator (I) added to the composition of the soap (s) and which has a turning area compatible with the pH of the latter.

3. Liquid antiseptic soap according to claim 2, characterised in that the coloured indicator is either phenol red or bromophenol red or bromothymol blue or the universal indicator.

4. Liquid antiseptic soap according to claim 1, characterised in that the colouring means are formed by a dyestuff (c) integrated into the composition of the liquid soap (s), which dyestuff is capable of changing the colour of this soap under the action of a reagent (R) after a determined period of washing.

5. Liquid antiseptic soap according to claim 4, characterised in that the reagent (R) is contained in different container than the one containing the liquid antiseptic soap (s).

6. Liquid antiseptic soap according to claim 5, characterised in that the reagent (R) is admixed either to the soap (s) and/or to a glycerine-containing softening solution (g) without dyestuff.

7. Liquid antiseptic soap according to claim 1, characterised in that the colouring means are in the form of microballs (M) capable of releasing a dyestuff after a determined period, through the adjunction of water to the soap, either through a simple dissolution phenomenon or under the action of a change in pH resulting from said adjunction of water.

8. Liquid antiseptic soap according to claim 7, characterised in that the microballs (M) are in the form of tiny balls (s') containing the dyestuff, these tiny balls (s') being coated with a film (e) formed of a product likely to become permeate under the action of a change in pH resulting from the adjunction of water to the soap or even capable of being progressively dissolved in this water.

9. Liquid antiseptic soap according to claim 7, characterised in that the microballs (M) are in the form of tiny balls (s) forming a neutral support coated with the active constituent (c) formed by the dyestuff, then coated with a film (e) formed of a product likely to become permeate under the action of a change in pH resulting from the adjunction of water to the soap or even capable of being progressively dissolved in this water.

10. Liquid antiseptic soap according to any of claims 8 or 9, characterised in that the coating (e) is carried out by means of cellulose acetylphthalate.

11. Liquid antiseptic soap according to claim 1, characterised in that the colouring means are in the form of liposomes forming capsules in which are inserted dyestuffs.
